Europäisches Patentamt

European Patent Office

Office européen des brevets

(19)

(11) Veröffentlichungsnummer : **0 182 118
B1**

(12) **EUROPÄISCHE PATENTSCHRIFT**

(45) Veröffentlichungstag der Patentschrift :
20.09.89

(21) Anmeldenummer : 85113324.9

(22) Anmeldetag : 21.10.85

(51) Int. Cl.⁴ : **C 07 C143/12, C 07 C139/14**

(54) **Pumpfähige hochkonzentrierte wässrige Pasten von Alkalisalzen Alpha-sulfonierter Fettsäurealkylester und Verfahren zu ihrer Herstellung.**

(30) Priorität : 29.10.84 DE 3439520

(43) Veröffentlichungstag der Anmeldung :
28.05.86 Patentblatt 86/22

(45) Bekanntmachung des Hinweises auf die Patenterteilung : 20.09.89 Patentblatt 89/38

(84) Benannte Vertragsstaaten :
AT BE CH DE FR IT LI NL SE

(56) Entgegenhaltungen :
EP–A– 0 054 724
DE–A– 3 319 591
DE–A– 3 334 517

(73) Patentinhaber : Henkel Kommanditgesellschaft auf Aktien
Postfach 1100 Henkelstrasse 67
D-4000 Düsseldorf-Holthausen (DE)

(72) Erfinder : Piorr, Robert, Dr.
Kieselei 12
D-4030 Ratingen-Hösel (DE)
Erfinder : Ritterbex, Horst
Beestrasse 44
D-4000 Düsseldorf 30 (DE)
Erfinder : Jost, Frantisek, Dr.
Bonner Strasse 14
D-4000 Düsseldorf 13 (DE)
Erfinder : Rommerskirchen, Hans Josef
Saganer Weg 21
D-4000 Düsseldorf (DE)

## Beschreibung

Alpha-Sulfofettsäureestersalze erhält man bekanntlich in Form wäßriger Pasten durch Neutralisation von Alpha-Sulfofettsäureestern mit insbesondere wäßrigem Alkalihydroxid. Als technisches Ausgangsmaterial dienen Fette und/oder Öle natürlichen Ursprungs, die durch Esterspaltung und nachfolgende Veresterung mit niederen Alkanolen insbesondere Methanol bzw. durch Umesterung der natürlichen Triglyceride mit den niederen Alkanolen erhalten werden. Die anfallenden Fettsäureestergemische enthalten — je nach Ursprung des natürlichen Rohstoffs — Fettsäuren eines vergleichsweise breiten Bereichs, der üblicherweise durch $C_{10}$ — bis $C_{24}$ — abgedeckt ist. Die Sulfonierung dieser Fettsäureestergemische, insbesondere mit gasförmigem $SO_3$, führt zu mehr oder weniger stark verfärbten sauren Rohsulfonaten, die gebleicht und durch Neutralisation auf den pH-Bereich von etwa 6 bis 7 in Estersulfonatpasten überführt werden müssen. In dieser Form haben sie heute zunehmende praktische Bedeutung als oberflächenaktive Mittel bzw. Netzmittel für Wasch- und Reinigungsmittel aus nachwachsenden Rohstoffen natürlichen Ursprungs.

Eine besondere Schwierigkeit in der technischen Handhabung solcher Pasten von Alkalisalzen Alpha-sulfonierter Fettsäurealkylester — im folgenden als Estersulfonatsalze bezeichnet — liegt in ihrem Konzentrations-/Viskositäts-Verhalten. Technisch anfallende Estersulfonatsalze bilden in wäßriger Abmischung nur bei vergleichsweise niedrigen Feststoffkonzentrationen — beispielsweise bis zu Feststoffgehalten von etwa 35 Gew.-% — hinreichend bewegliche Lösungen bzw. Suspensionen um den störungsfreien Ablauf technischer Vorgänge zu gewährleisten. Bei höheren Estersulfonatsalzgehalten — Feststoffgehalte von etwa 40 Gew.-% oder mehr — steigt die Viskosität der wäßrigen Zubereitung so stark an, daß ihre freie Beweglichkeit nicht mehr besteht. Hieraus resultieren schwerwiegende Einschränkungen :

Der Versuch, unmittelbar hochkonzentrierte Estersulfonatsalzpasten durch Neutralisation des Rohsulfonsäuregemisches mit konzentrierter Alkalihydroxidlösung zu gewinnen mißlingt, weil die Rührbarkeit und damit die gleichmäßige Vermischbarkeit des Reaktionsgemisches verloren geht. Gleichzeitig damit wird der Abtransport der Neutralisationswärme unmöglich. Durch lokale Konzentrations- und Temperaturspitzen treten unerwünschte Nebenreaktionen — insbesondere eine unerwünscht hohe Bildung von Disalzen der Alpha-Sulfofettsäuren unter Esterspaltung — ein. Nachteilig ist begreiflicherweise auch, daß durch Viskositätsanstieg immobilisierte Estersulfonatpasten im großtechnischen Betrieb nicht mehr pumpbar sind. Es tritt der Verschluß von Rohrleitungen und damit eine nachhaltige Störung des Betriebs der Gesamtanlage ein.

Der Stand der Technik zu Estersulfonatsalzpasten der hier betroffenen Art beschäftigt sich intensiv mit diesen Besonderheiten. Vorgeschlagen wurde insbesondere, zur Verbesserung des Fließverhaltens von wäßrigen technischen Konzentraten von Alpha-Sulfofettsäureestersalzen Fließhilfsmittel bzw. Viskositätsregler einzusetzen. So schildert beispielsweise die DE-OS 33 05 430 die Mitverwendung von $C_8$-$C_{40}$-Alkoholen, die zusätzlich eine oder mehrere Hydroxylgruppen als Substituenten aufweisen können und an die bis zu 20 Mol Ethylenoxid und/oder Propylenoxid pro Mol Alkanol angelagert sein können. Diese Viskositätsregler werden der Estersulfonatpaste in Mengen von 1 bis 15 Gew.-%, bezogen auf die Tensidmenge, zugefügt, wobei sich eine Viskosität des Tensidkonzentrates von höchstens 10 000 mPas bei 70 °C einstellt. Nach der Lehre der DE-OS 33 34 517 sollen wäßrige Aufschlämmungen hinreichend beweglich sein, die 40 bis 65 Gew.-% Alpha-Sulfofettsäureestersalz, 2 bis 10 Gew.-% eines niedrigen Alkoholsulfats und gegebenenfalls höchstens 2 Gew.-% eines niedrigen Alkohols enthalten. Diese wäßrigen Mehrstoffgemische werden dadurch erhalten, daß man zunächst wäßrige Aufschlämmungen mit 30 bis 55 Gew.-% des Alpha-Sulfofettsäureestersalzes in Abmischung mit 5 bis 15 Gew.-% eines niedrigen Alkoholsulfats und 8 bis 40 Gew.-% eines niedrigen Alkohols herstellt und dann die wäßrige Aufschlämmung auf die zuvor genannten Konzentrationswerte einengt. Die DE-OS 31 23 681 will zur Herstellung einer hochkonzentrierten wäßrigen Lösung eines Salzes eines Alpha-Sulfofettsäureesters in einer ersten Stufe die Rohsulfonsäure mit einer 15 bis 50 gewichtsprozentigen wäßrigen kaustischen Alkalilösung in Gegenwart von 5 bis 20 Gew.-% eines Alkohols mit bis zu 4 Kohlenstoffatomen auf einen pH-Wert von 2,5 bis 4 neutralisieren, woraufhin in einer nachfolgenden Stufe zu dem noch sauren teil-neutralisierten Produkt eine auf 1 bis 5 Gew.-% Alkalihydroxid verdünnte wäßrige Lösung zugesetzt und der pH-Wert von 6 bis 7 eingestellt wird.

Alle diese Vorschläge des Standes der Technik beziehen sich auf die üblichen-technischen Einsatzmaterialien natürlichen Ursprungs mit der vergleichsweise großen Breite zur C-Zahl im vorliegenden Fettsäuregemisch von beispielsweise $C_{10}$-$C_{24}$. Die-Erfindung geht von der überraschenden Feststellung aus, daß bei der Auswahl ganz bestimmter Kettenlängen im Fettsäureanteil der Estersulfonatsalze Anomalien im Viskositätsverhalten hochkonzentrierter Salzpasten dergestalt auftreten, daß nach dem zunächst üblichen Anstieg der Viskositätswerte beim Ansteigen des Feststoffgehaltes über etwa 40 Gew.-% hinaus schließlich ein Feststoffgehalt erreicht wird, in dem die Viskositätswerte der hochkonzentrierten wäßrigen Estersulfonatsalzpasten wieder so drastisch absinken, daß ihre freie Beweglichkeit und insbesondere Pumpbarkeit bei üblichen Verarbeitungstemperaturen gewährleistet sind.

Gegenstand der Erfindung sind dementsprechend in einer ersten Ausführungsform bei Temperaturen von wenigstens 60 °C pumpfähige wäßrige Pasten mit wenigstens 40 Gew.-% an Alkalisalzen Alpha-sulfonierter Fettsäurealkylester (Estersulfonatsalze), die dadurch gekennzeichnet sind, daß sie im wesentlichen Estersulfonatsalze auf Basis von $C_{16}$- und/oder $C_{18}$-Fettsäuren enthalten, Estersulfonatsalz-Feststoffgehalte von wenigstens etwa 60 Gew.-%, vorzugsweise von wenigstens etwa 65 Gew.-%, aufweisen und weiterhin praktisch frei sind von Viskositätsreglern oder anderen die Viskosität senkenden Zusatzstoffen.

Die Lehre der Erfindung geht damit von der überraschenden Erkenntnis aus, daß bei der Einschränkung des Fettsäureanteils auf den Bereich $C_{16}/C_{18}$ im Estersulfonatsalz für wäßrige Aufschlämmungen eines besonders hohen Konzentrationsbereiches, der insbesondere im Bereich von etwa 60 bis 80 Gew.-%, vorzugsweise im Bereich von etwa 65 bis 80 Gew.-%, liegt bei nur mäßig erhöhten Temperaturen von beispielsweise 40 bis 70 °C die freie Pumpbarkeit und Vermischbarkeit dieser Massen gewährleistet ist, während beim Absenken des Feststoffgehaltes der gleichen Estersulfonatsalze etwa auf den Konzentrationsbereich von 40 bis 60 Gew.-% das bisher bekannte Konzentrations-Viskositätsverhalten vorliegt — d. h. die entsprechenden wäßrigen Stoffgemische nicht pumpbar bzw. beweglich sind. Das gleiche trifft zu, wenn die erfindungsgemäße Einschränkung bezüglich der Kohlenstoffzahl im Fettsäureanteil der Estersulfonatsalze nicht eingehalten wird und beispielsweise übliche technische Ausgangsmaterialien auf Basis von Fettsäuregemischen des C-Zahlbereichs von 12 bis 18 Verwendung finden. Für solche Estersulfonatsalzpasten liegt im erhöhten Feststoffbereich von 60 bis 80 Gew.-% kein Abfall der Viskosität vor, der ihre freie und sichere Handhabung ohne Zusatz von Viskositätsreglern oder anderen vorbekannten Maßnahmen ermöglicht.

Geringfügige Beimischungen von Fettsäuren bzw. Fettsäureestern des unerwünschten C-Zahlbereichs, wie sie bei der technischen Aufarbeitung von natürlichen Fettsäuregemischen anfallen können, müssen nicht notwendigerweise störend sein. Erfindungsgemäß brauchbare Estersulfonatsalzpasten enthalten jedoch solche vom $C_{16}/C_{18}$-Bereich abweichende Fettsäuren und insbesondere kürzerkettige Fettsäureester höchstens in Mengen bis zu etwa 10 Molprozent, bevorzugt liegen solche begleitenden Anteile deutlich unter 10 Molprozent. Die Mischungsverhältnisse von $C_{16}$- zu $C_{18}$-Fettsäuren können bevorzugt etwa im Bereich von 3 : 2 bis 0 : 1 liegen, wobei wiederum dem Bereich von etwa 3 : 2 bis 1 : 2 besondere Bedeutung zukommen kann. Ein besonders wichtiges Ausgangsmaterial für die Gewinnung der erfindungsgemäß eingestellten hochkonzentrierten und gleichwohl pumpfähigen Estersulfonatsalzpasten sind Fettsäureester — insbesondere Fettsäuremethylester — die ein Mischungsverhältnis von $C_{16}$ — zu $C_{18}$ — etwa im Bereich von 1 : 1 aufweisen.

Technische Ausgangsmaterialien mit den geforderten Mischungsverhältnissen für die Zahl der Kohlenstoffatome im Fettsäureanteil fallen bei bestimmten Aufarbeitungsprozessen für natürliche Fette und/oder Öle an. So führt die Gewinnung von Ölsäure durch Spaltung von Talgfett nach dem sogenannten Umnetzverfahren oder die Aufarbeitung von Palmöl zu einem Produkt, das die $C_{16}$- und $C_{18}$-Fettsäuren etwa im Verhältnis von 1 : 1 enthält. Hier ist somit ein besonders geeignetes Ausgangsmaterial für die Verwirklichung der erfindungsgemäßen Lehre gegeben. Die Gewinnung des sogenannten Palmstearins aus Palmöl führt zu Fettsäure- bzw. Fettsäureestergemischen mit einem $C_{16}/C_{18}$-Verhältnis von etwa 60/40. Die im praktischen Verfahren der Talgaufbereitung anfallenden Fettsäureschnitte liegen häufig im $C_{16}/C_{18}$-Verhältnis von 1 : 2 mit nur geringfügigen Verunreinigungen durch andere Fettsäuren. Gehärtetes Sojaöl enthält ein $C_{16}/C_{18}$-Mischungsverhältnis von etwa 8 : 92. Auch diese Ausgangsmaterialien sind für die Verwirklichung der erfindungsgemäßen Lehre geeignet. Andere übliche Fett- bzw. Ölrohstoffe mit Fettsäuremischungen, beispielsweise des Bereichs $C_{12}$ bis $C_{18}$ und gleichzeitig nur beschränkter Menge an Fettsäuren des Bereichs $C_{16}$-$C_{18}$ geben demgegenüber bei der Überführung in Estersulfonatsalzpasten eines Feststoffgehalts von 50 Gew.-% und mehr keine niederen Viskositäten.

Der pH-Wert der erfindungsgemäßen hochkonzentrierten wäßrigen Estersulfonatsalzpasten liegt vorzugsweise im Bereich von etwa 6 bis 7. Die durch Rotationsviskosimeter bestimmte Viskosität der Pasten liegt bei 70 °C im allgemeinen nicht über etwa 10 000 mPas. Der Gehalt an Disalzen bestimmt sich primär durch die gewählten Ansatzverhältnisse in der Stufe der Estersulfonierung. Bekanntlich wird hier üblicherweise mit einem etwa 20 prozentigen Überschuß an $SO_3$ gearbeitet, was bei einer Neutralisation ohne zusätzliche Maßnahmen zu einem Disalzgehalt von etwa 20 bis 25 Gew.-%, bezogen auf Waschaktivsubstanz (WAS), führt. Disalzgehalte dieser Größenordnung stören das angestrebte Viskositätsverhalten der hochkonzentrierten Estersulfonatsalzpasten nicht. Der Disalzgehalt kann aber gewünschtenfalls auch eingeschränkt werden, hierzu lassen sich bekannte Maßnahmen des Standes der Technik, insbesondere aber auch der Vorschlag der älteren Anmeldung gemäß P 34 32 324.4 « Verfahren zur Regelung des Disalzgehaltes in Alpha-Sulfofettsäureester-Tensiden (D 7116) einsetzen.

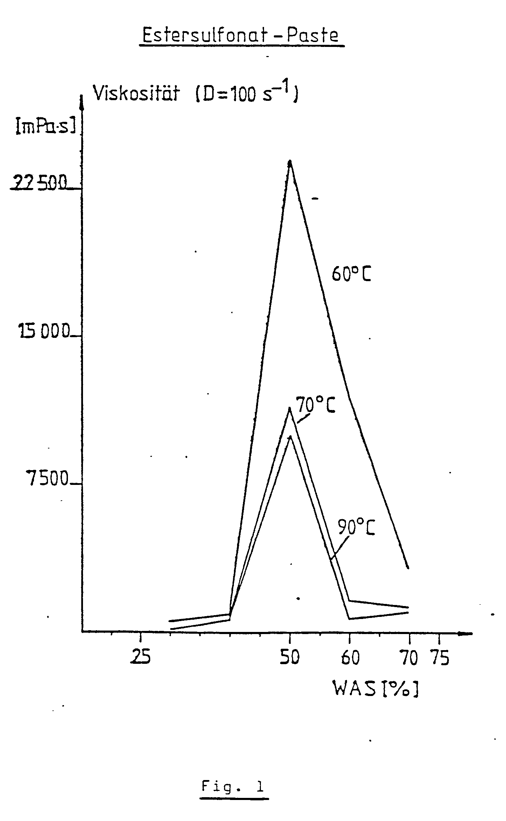

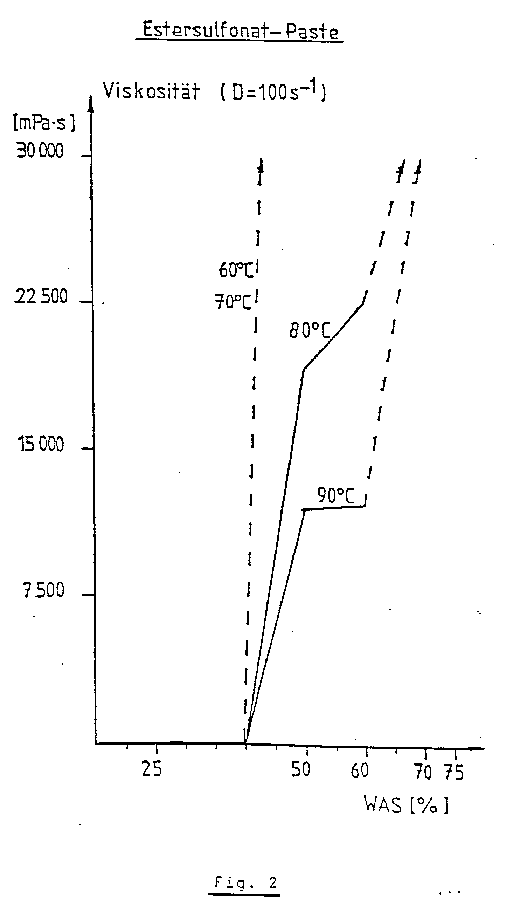

In der beigefügten Figur 1 ist die Abhängigkeit des Viskositätsverhaltens einer erfindungsgemäßen Estersulfonatsalzpaste vom Feststoffgehalt (Prozent WAS) dargestellt, wobei gleichzeitig die Temperaturabhängigkeit im Bereich von 60 bis 90 °C ersichtlich wird. Das hier untersuchte Estersulfonatsalz enthält Alpha-sulfonierte Fettsäuremethylester mit einem Mischungsverhältnis von $C_{16}/C_{18}$ von etwa 1 : 1 wie es als Kuppelprodukt bei der Spaltung von Talgfett durch Umnetzverfahren anfällt. Figur 1 zeigt, daß beginnend mit einem Feststoffgehalt von 40 % WAS ein steiler Anstieg der Viskosität — Scherviskosität ($D = 100$ $s^{-1}$) — eintritt, der bei ca. 50 % WAS sein Maximum findet. Bei weiterer Steigerung des Feststoffgehaltes in der Paste sinkt die Viskosität und erreicht zwischen 60 und 75 % WAS-Werte, die etwa dem Viskositätsverhalten einer 30 bis 40 %igen Estersulfonatsalzpaste entsprechen. Demgegenüber zeigt die beigefügte Figur 2 die Abhängigkeit des Viskositätsverhaltens einer Estersulfonatsalzpaste, die sich aus einem handelsüblichen Palmkernöl mit einem $C_{12}$- bis $C_{18}$-Fettsäureschnitt ableitet, wobei der $C_{12}$- bis $C_{14}$-Fettsäureanteil wenigstens etwa 70 % ausmacht. Auch hier findet, beginnend bei etwa 40 % WAS, ein steiler Anstieg der Viskosität statt, beim Überschreiten der Feststoffgehaltsgrenzen von 50 bzw. 60 % WAS tritt jedoch kein Abfall der Viskosität auf.

Die der Erfindung zugrunde liegende Erkenntnis führt nicht nur zur Möglichkeit, ausgewählte Estersulfonatsalzpasten vor Feststoffkonzentrationen sicher handhaben zu können, wichtig ist die Lehre der Erfindung insbesondere schon für die Herstellung der Estersulfonatsalzpasten hoher Konzentration. Die bisher als unvermeidbar angesehenen Schwierigkeiten bei der Neutralisationsreaktion und damit auftretenden unerwünschten Eindickungserscheinungen — und dementsprechend Reaktionsinhomogenitäten mit Nebenreaktionen — lassen sich erfindungsgemäß sicher bewältigen. In der im folgenden geschilderten Ausführungsform der Erfindung können unmittelbar Estersulfonatpasten hohen Feststoffgehaltes durch Neutralisation der Rohsulfonsäuren mit hochkonzentrierten Alkalihydroxidlösungen, insbesondere mit hochkonzentriertem Natriumhydroxid erhalten werden. Gegenstand der Erfindung ist dementsprechend in dieser weiteren Ausführungsform das Verfahren zur Herstellung der hochkonzentrierten Estersulfonatsalzpasten, wobei dieses Verfahren dadurch gekennzeichnet ist, daß man die Esterrohsulfonate — gegebenenfalls nach einer an sich bekannten sauren Bleiche, insbesondere mit $H_2O_2$ — mit konzentrierter wäßriger Alkalihydroxidlösung unter intensiver Kühlung bei Temperaturen unterhalb 90 °C zu Estersulfonatsalzpasten eines Feststoffgehaltes von wenigstens etwa 60 Gew.-% auf den pH-Bereich von etwa 6 bis 7 neutralisiert und gewünschtenfalls einer weiteren Bleichstufe — insbesondere mit NaOCl — unterwirft. Bevorzugt werden dabei die Esterrohsulfonate und die konzentrierte wäßrige Alkalihydroxidlösung in eine intensiv bewegte und gekühlte Estersulfonatpaste hohen Feststoffgehalts — bevorzugt wenigstens etwa 60 Gew.-% WAS — eingespeist, so daß die Neutralisation unter Salzbildung im Schoße der angestrebten vergleichsweise niedrig viskosen wäßrigen Estersulfonatsalzpaste erfolgt.

Wichtig ist bei der Durchführung dieses Neutralisationsverfahrens die hinreichende Kühlung der Reaktionsmischung zur Abführung der Neutralisationswärme, so daß ein oberer Temperaturbereich von etwa 90 °C nicht oder nicht wesentlich überschritten wird. Geeignet sind insbesondere Neutralisationstemperaturen im Bereich von etwa 60 bis 90 °C. Es ist weiterhin bevorzugt die Verweilzeit — in kontinuierlichen Verfahren die mittlere Verweilzeit — des Reaktionsmediums im angegebenen Temperaturbereich zu begrenzen. Bevorzugt liegt die Verweilzeit bzw. mittlere Verweilzeit nicht über etwa 30 Minuten und insbesondere unter etwa 20 Minuten. Durch die Einhaltung dieser Parameter von Temperatur und Verweilzeit wird bei gleichzeitig intensiver Vermischung der Masse, die durch die geschilderte niedere Viskosität ermöglicht wird, die homogene Einmischung der Reaktanten Alkalihydroxid und Rohsulfonsäure in die Estersulfonatpaste sichergestellt und damit das Auftreten von örtlichen Konzentrations- und/oder Temperaturunterschieden ausgeschlossen. Dadurch, daß von vornherein der WAS-Feststoffgehalt des Reaktionsmediums auf den Bereich so hoher Konzentrationen gesetzt wird, daß die erforderliche niedrige Viskosität vorliegt, werden darüber hinaus Störungen durch zwischenzeitliche Immobilisierung des Reaktionsgemisches aufgrund zu hoher Viskositäten ausgeschlossen.

Mit der erfindungsgemäßen Verfahrensvariante können — gegebenenfalls vorgebleichte — Rohsulfonate unmittelbar mit wäßrigen Alkalihydroxidlösungen eines Konzentrationsbereichs von beispielsweise 15 bis 70 Gew.-% und vorzugsweise 30 bis 55 Gew.-% in leicht bewegliche vollneutralisierte Estersulfonatsalzpasten des pH-Bereichs von 6 bis 7 überführt werden. Im einzelnen lassen sich dabei verschiedene Verfahrensmodifikationen vorsehen. In einer bevorzugten Ausführungsform wird die Neutralisation kontinuierlich in einer Reaktionsschleife durchgeführt, durch die eine erfindungsgemäße $C_{16}/C_{18}$-Estersulfonatsalzpaste mit Feststoffgehalten von wenigstens 60 Gew.-% im Kreislauf geführt und gekühlt wird. In diese Reaktionsschleife wird einerseits Alpha-sulfonierter Fettsäureester des angegebenen C-Zahlbereiches und wäßriges Neutralisationsmittel eingeschleust, andererseits wird ein entsprechender Anteil an Estersulfonatpaste ausgeschleust. Die Temperatur in der Neutralisationsstufe wird beispielsweise im Bereich von 80 bis 90 °C gehalten. Wird die Rohsulfonsäure vor der Neutralisation einer an sich bekannten sauren $H_2O_2$-Bleiche unterworfen, so kann es zweckmäßig sein, die Gutführung im Neutralisationskreislauf unter Drucken — beispielsweise Drucke bis zu 10 bar — vorzunehmen. Die ausgeschleusten Anteile an Estersulfonatpaste können gewünschtenfalls einer Nachbleiche mit NaOCl im Neutralbereich unterworfen werden.

Anstelle der Umsetzung von Alkalihydroxid und Rohsulfonat im geschilderten Neutralisationskreislauf kann aber auch die Neutralisation in intensiv gerührten und gekühlten Reaktionsbehältern erfolgen, die bereits hinreichende Mengen an Estersulfonatpaste enthalten. Hier kann absatzweise oder auch kontinuierlich gearbeitet werden.

Viskositätsprobleme, die zu Beginn eines kontinuierlichen Verfahrens auftreten, lassen sich auf die folgende Weise leicht bewältigen :

Zu Beginn der Neutralisationsreaktion wird dem Reaktionsgemisch ein Viskositätsregler zugemischt und gleichzeitig mit hinreichend verdünnten Reaktionslösungen gearbeitet, so daß mit Sicherheit das Auftreten von unerwünschten Immobilisierungerscheinungen verhindert wird. Derartig niedrig konzentrierte und/oder Viskositätsregler enthaltende Estersulfonatsalzpasten werden unter kontinuierlicher Einspeisung von zusätzlichem Viskositätsregler im Estersulfonatsalzgehalt aufkonzentriert. Die Viskosität wird dabei zweckmäßigerweise stets bei Werten von höchstens etwa 10 000 mPas bei 70 °C gehalten. Nach Erreichen des Viskositätsmaximums im Bereich von etwa 50 Gew.-% WAS-Gehalt fällt bei weiterer Feststofferhöhung die Viskosität. Jetzt kann die Zugabe von Viskositätsregler erniedrigt werden bis im erwünschten Konzentrationsbereich der Estersulfonatsalzpaste auf zusätzliches Einschleusen von Viskositätsreglern vollständig verzichtet werden kann. Gleichzeitig wird durch die Einspeisung hochkonzentrierter Reaktanten, (insbesondere hochkonzentrierte wäßrige Alkalihydroxidlösung) dafür Sorge getragen, daß der Feststoffgehalt des Reaktionsmediums im gewünschten Bereich liegt.

Beispiel

In einer kontinuierlichen Sulfieranlage werden pro Stunde 42,5 kg ( = 150 Mol) gehärteter Talgfettsäuremethylester ($C_{16}/_{18}$ 1 : 1), dem vor der Härtung der Ölsäureanteil durch Trennung nach dem Umnetzverfahren entzogen worden ist, mit 14,4 kg $SO_3$/Stunde ( = 180 Mol) verdünnt auf 5 Vol-% mit trockener Luft zu alpha-Sulfo-Talgmethylester umgesetzt.

Dieses Sulfonierungsgemisch wird bei 80 °C einer, zu Beginn mit Wasser gefüllten, kontinuierlichen Neutralisationsstufe zugeführt und gleichzeitig 50 %ige Natronlauge so zudosiert, daß ein pH-Wert von 5 bis 7 erreicht wird. Durch Kühlung wird der Temperaturbereich von 80 bis 90 °C eingehalten. Das neutralisierte Produkt wird im Kreislauf zurückgeführt und auf diese Weise aufkonzentriert. Ab ca. 25 Gew.-% Aktivsubstanz (AS) im neutralisierten Produkt werden zusätzlich 5 bis 8,5 kg $C_{28}$-Guerbetalkohol/Stunde (10 bis 15 %, bezogen auf AS) als Viskositätserniedriger zudosiert, bis eine Konzentration von 55 bis 60 % AS erreicht ist. Danach wird die Zugabe von Guerbetalkohol stufenweise gedrosselt bis auf 0 % und das neutralisierte Produkt auf ca. 70 % AS eingestellt. Die hochkonzentrierte Paste ist im Temperaturbereich 60 bis 90 °C ohne Schwierigkeiten pumpbar.

**Patentansprüche**

1. Bei Temperaturen von wenigstens 60 °C pumpfähige wäßrige Pasten mit wenigstens 40 Gew.-% an Alkalisalzen Alpha-sulfonierter Fettsäurealkylester (Estersulfonatsalze) dadurch gekennzeichnet, daß sie im wesentlichen Estersulfonatsalze auf Basis von $C_{16}$- und/oder $C_{18}$-Fettsäuren enthalten, Estersulfonatsalz-Feststoffgehalte von wenigstens 60 Gew.-% aufweisen und praktisch frei von Viskositätsreglern sind.

2. Estersulfonatsalzpasten nach Anspruch 1, dadurch gekennzeichnet, daß ihr Gehalt an Estersulfonatsalzen von Fettsäuren mit weniger als 16 C-Atomen weniger als 10 Mol-Prozent beträgt.

3. Estersulfonatsalzpasten nach den Ansprüchen 1 und 2, dadurch gekennzeichnet, daß ihr Mischungsverhältnis von $C_{16}$- zu $C_{18}$-Fettsäuren im Bereich von 3 : 2 bis 0 : 1 liegt und beispielsweise 3 : 2 bis 1 : 2 ausmachen kann.

4. Estersulfonatsalzpasten nach den Ansprüchen 1 bis 3, dadurch gekennzeichnet, daß sie sich von technischen Fettstoffen natürlichen Ursprungs ableiten und wenigstens überwiegend Alpha-sulfonierte Fettsäuremethylestersalze enthalten.

5. Estersulfonatsalzpasten nach den Ansprüchen 1 bis 4, dadurch gekennzeichnet, daß sie bei pH-Werten im Bereich von 6 bis 7 und Temperaturen von 60 bis 70 °C Viskositätswerte nicht über 10 000 mPas und vorzugsweise Gehalte an Disalzen nicht über 25 Gew.-%/WAS aufweisen.

6. Verfahren zur Herstellung der Estersulfonatsalzpasten nach den Ansprüchen 1 bis 5, dadurch gekennzeichnet, daß man die Esterrohsulfonate — gegebenenfalls nach einer an sich bekannten sauren Bleiche mit $H_2O_2$ — mit konzentrierter wäßriger Alkalihydroxidlösung unter intensiver Kühlung bei Temperaturen nicht über 90 °C zu Estersulfonatsalzpasten eines Feststoffgehalts von wenigstens 60 Gew.-% — vorzugsweise von 65 bis 80 Gew.-% — auf den pH-Bereich von 6 bis 7 neutralisiert und gewünschtenfalls einer weiteren Bleichstufe — insbesondere mit NaOCl — unterwirft.

7. Verfahren nach Anspruch 6, dadurch gekennzeichnet, daß die Esterrohsulfonate und konzentrierte wäßrige Alkalihydroxidlösung · in eine intensiv bewegte und gekühlte Estersulfonatsalzpaste eines Feststoffgehalts von mindestens 80 Gew.-% eingespeist werden, wobei bevorzugt die mittlere Verweilzeit dieses Reaktionsgemisches unter den Temperaturbedingungen der Neutralisationsreaktion nicht mehr als 20 Minuten beträgt.

8. Verfahren nach den Ansprüchen 6 und 7, dadurch gekennzeichnet, daß mit wäßrigen Alkalihydroxidlösungen — insbesondere wäßriger Natronlauge — eines Konzentrationsbereichs von 15 bis 50 Gew.-% gearbeitet wird.

9. Verfahren nach den Ansprüchen 6 bis 8, dadurch gekennzeichnet, daß man die Neutralisation kontinuierlich in einer Reaktionsschleife durchführt, durch die die Estersulfonatsalzpaste mit Feststoffgehalten von wenigstens 60 Gew.-% im Kreislauf geführt und gekühlt wird, während einerseits Alpha-sulfonierter Fettsäureester und wäßriges Neutralisationsmittel eingeschleust und andererseits ein entsprechender Anteil an Estersulfonatsalzpaste ausgeschleust werden.

10. Verfahren nach den Ansprüchen 6 bis 9, dadurch gekennzeichnet, daß man in kontinuierlicher Arbeitsweise zu Beginn der Neutralisationsreaktion dem Reaktionsgemisch einen Viskositätserniedriger und Wasser zumischt, wobei man zunächst Viskositätserniedriger enthaltende wäßrige Konzentrate mit Gehalten an Alpha-Sulfofettsäureestersalzen unterhalb 60 Gew.-% und einer Viskosität von höchstens 10 000 mPas bei 70 °C einstellt, unter Kühlung und Kreislaufführung allmählich die Zugabe von Viskositätserniedriger und Wasser verringert, bis ohne Zumischung von Viskositätserniedriger und Wasser als Reaktionsprodukt eine Paste mit 60 bis 80 Gew.-% Alpha-Sulfofettsäureestersalz anfällt.

**Claims**

1. Aqueous pastes pumpable at temperatures of at least 60 °C containing at least 40 % by weight alkali salts of α-sulfonated fatty acid alkyl esters (ester sulfonate salts), characterized in that they essentially contain ester sulfonate salts based on $C_{16}$ and/or $C_{18}$ fatty acids, have ester sulfonate salt solids contents of at least 60 % by weight and are substantially free from viscosity regulators.

2. Ester sulfonate salt pastes as claimed in Claim 1, characterized in that their content of ester sulfonate salts of fatty acids containing less than 16 carbon atoms amounts to less than 10 mole %.

3. Ester sulfonate salt pastes as claimed in Claims 1 and 2, characterized in that the ratio of $C_{16}$ to $C_{18}$ fatty acids therein is from 3 :2 to 0 :1 and may amount, for example, to between about 3 :2 and 1 :2.

4. Ester sulfonate salt pastes as claimed in Claims 1 to 3, characterized in that they are derived from commercial fats of natural origin and, at least for the most part, contain α-sulfonated fatty acid methyl ester salts.

5. Ester sulfonate salt pastes as claimed in Claims 1 to 4, characterized in that they have viscosities not exceeding 10,000 mPas at pH-values of from 6 to 7 and at temperatures of from 60 to 70 °C and preferably contain no more than 25 % by weight, based on WAS, of disalts.

6. A process for producing the ester sulfonate salt pastes claimed in Claims 1 to 5, characterized in that, optionally after acidic bleaching in known manner with $H_2O_2$, the crude ester sulfonates are neutralized with intensive cooling at temperatures not exceeding 90 °C to a pH-value of from 6 to 7 using concentrated aqueous alkali hydroxide solution to form ester sulfonate salt pastes having a solids content of at least 60 % by weight and preferably of from 65 to 80 % by weight and, if desired, are subjected to another bleaching treatment, more especially with NaOCl.

7. A process as claimed in Claim 6, characterized in that the crude ester sulfonates and concentrated aqueous alkali hydroxide solution are introduced into an intensively stirred and cooled ester sulfonate salt paste having a solids content of at least 60 % by weight, the average residence time of this reaction mixture under the temperature conditions of the neutralization reaction preferably being no more than 20 minutes.

8. A process as claimed in Claims 6 and 7, characterized in that aqueous alkali hydroxide, particularly sodium hydroxide, solutions having a concentration of from 15 to 50 % by weight are used.

9. A process as claimed in Claims 6 to 8, characterized in that neutralization is carried out continuously in a reaction loop through which the ester sulfonate salt paste having solids contents of at least 60 % by weight is circulated and cooled, whilst on the one hand α-sulfonated fatty acid esters and aqueous neutralizing agent are introduced into the loop and, on the other hand, a corresponding proportion of ester sulfonate salt paste is removed therefrom.

10. A process as claimed in Claims 6 to 9, characterized in that, where it is carried out continuously, a viscosity reducing agent and water are added to the reaction mixture at the beginning of the neutralization reaction, viscosity-reduced aqueous concentrates containing less than 60 % by weight of α-sulfofatty acid ester salts and having a viscosity of at most 10,000 mPas at 70 °C initially being obtained, after which the addition of viscosity-reducing agent and water is gradually reduced with cooling and circulation until a paste containing from 60 to 80 % by weight of α-sulfofatty acid ester salt is obtained as the reaction product without the addition of viscosity-reducing agent and water.

**Revendications**

1. Pâtes aqueuses aptes au pompage à des températures d'au moins 60 ºC, contenant au moins 40 % en poids de sels alcalins d'esters alkyliques d'acides gras alpha-sulfonés (sels de sulfonates d'esters), caractérisées en ce qu'elles contiennent essentiellement des sels de sulfonates d'esters à base d'acides gras en $C_{16}$ et/ou $C_{18}$, présentent des teneurs en matière sèche de sels de sulfonates d'esters d'au moins 60 % en poids, et sont pratiquement exemptes de régulateurs de viscosité.

2. Pâtes de sels de sulfonates d'esters selon la revendication 1, caractérisées en ce que leur teneur en sels de sulfonates d'esters d'acides gras ayant moins de 16 atomes de carbone est inférieure à 10 % en mole.

3. Pâtes de sels de sulfonates d'esters selon la revendication 1 ou 2, caractérisées en ce que le rapport de mélange d'acides gras en $C_{16}$ à acides gras en $C_{18}$ est dans la plage de 3 :2 à 0 :1 et peut représenter par exemple de 3 :2 à 1 :2.

4. Pâtes de sels de sulfonates d'esters selon l'une quelconque des revendications 1 à 3, caractérisées en ce qu'elles dérivent de matières grasses industrielles d'origine naturelle et contiennent au moins en majorité des sels d'esters méthyliques d'acides gras alpha-sulfonés.

5. Pâtes de sels de sulfonates d'esters selon l'une quelconque des revendications 1 à 4, caractérisées en ce qu'elles présentent, à des pH dans la plage de 6 à 7 et à des températures de 60 à 70 °C, des indices de viscosité n'excédant pas 10 000 mPa·s et de préférence des teneurs en disels n'excédant pas 25 % en poids/SAL (substance active lessivielle).

6. Procédé pour la préparation des pâtes de sels de sulfonates d'esters selon l'une quelconque des revendications 1 à 5, caractérisé en ce que l'on neutralise à un pH dans la plage de 6 à 7 les sulfonates bruts d'esters — éventuellement après un blanchiment acide avec $H_2O_2$ connu en soi — à l'aide d'une solution aqueuse concentrée d'un hydroxyde alcalin, avec intense refroidissement, à des températures n'excédant pas 90 °C, pour l'obtention de pâtes de sels de sulfonates d'esters ayant une teneur en matière sèche d'au moins 60 % en poids, de préférence de 65 à 80 % en poids, et, si on le désire, on les soumet à une nouvelle étape de blanchiment — en particulier à l'aide de NaOCl.

7. Procédé selon la revendication 6, caractérisé en ce que l'on introduit les sulfonates bruts d'esters et une solution aqueuse concentrée d'hydroxyde alcalin dans une pâte de sels de sulfonates d'esters vigoureusement agitée et fortement refroidie, ayant une teneur en matière sèche d'au moins 60 % en poids, le temps de séjour moyen de ce mélange réactionnel dans les conditions de température de la réaction de neutralisation n'excédant de préférence pas 20 minutes.

8. Procédé selon la revendication 6 ou 7, caractérisé en ce que l'on opère avec des solutions aqueuses d'hydroxyde alcalin — en particulier une solution aqueuse d'hydroxyde de sodium — ayant une concentration dans la plage de 15 à 50 % en poids.

9. Procédé selon l'une quelconque des revendications 6 à 8, caractérisé en ce que l'on effectue la neutralisation en continu dans une boucle de réaction dans laquelle on met en circuit et refroidit la pâte de sels de sulfonates d'esters ayant une teneur en matière sèche d'au moins 60 % en poids, tandis que d'une part on introduit des esters d'acides gras alpha-sulfonés et l'agent aqueux de neutralisation, et d'autre part on évacue une quantité correspondante de pâte de sels de sulfonates d'esters.

10. Procédé selon l'une quelconque des revendications 6 à 9, caractérisé en ce que, dans un mode opératoire en continu, au début de la réaction de neutralisation, on ajoute au mélange réactionnel un réducteur de viscosité et de l'eau, en ajustant d'abord des concentrés aqueux contenant un réducteur de viscosité, à teneurs en sels d'esters d'acides gras alpha-sulfonés inférieures à 60 % en poids et ayant une viscosité d'au maximum 10 000 mPa·s à 70 °C, en refroidissant et en mettant le mélange en circuit, on diminue progressivement l'addition de réducteur de viscosité et d'eau, jusqu'à ce que se forme en tant que produit de réaction, sans addition de réducteur de viscosité, ni d'eau, une pâte contenant de 60 à 80 % en poids de sel d'ester d'acide gras alpha-sulfoné.

Estersulfonat - Paste

Fig. 1

Estersulfonat–Paste

Viskosität ( D=100s⁻¹)

Fig. 2